# EUROPÄISCHE PATENTSCHRIFT

(11) **EP 0 775 156 B1**
(45) Veröffentlichungstag und Bekanntmachung des Hinweises auf die Patenterteilung: **03.09.2003**
(21) Anmeldenummer: 95925680.1
(22) Anmeldetag: 03.07.1995
(51) Int. Cl.: C07J 41/00, A61K 31/56, C07J 53/00, C07J 43/00

(54) **PHARMAZEUTISCHE ZUSAMMENSETZUNGEN MIT ESTRA-1,3,5(10)-TRIEN-DERIVATEN**
PHARMACEUTICAL COMPOSITIONS CONTAINING ESTRA-1,3,5(10)-TRIENE DERIVATIVES
COMPOSITIONS PHARMACEUTIQUES COMPRENANT DES DERIVES ESTRA-1,3,5(10)-TRIENE

(30) Priorität: 09.08.1994 DE 4429398
(43) Veröffentlichungstag der Anmeldung: 28.05.1997
(73) Patentinhaber: Jenapharm GmbH & Co. KG, 07745 Jena (DE)
(72) Erfinder: SIEMANN, Hans-Joachim, verstorben (DE); ELGER, Walter, D-14195 Berlin (DE); SCHWARZ, Sigfrid, D-07743 Jena (DE); REDDERSEN, Gudrun, D-07749 Jena (DE); SCHNEIDER, Birgitt, D-07745 Jena (DE)
(74) Vertreter: Ziebig, Marlene, Dr. Dipl.-Chem.
(86) Internationale Anmeldenummer: DE9500879
(87) Internationale Veröffentlichungsnummer: WO96005217

(56) Entgegenhaltungen:
- EP-A- 0 430 386
- WO-A-94/01450
- DD-A- 114 806
- DD-A- 207 447
- DD-A1- 201 143
- DE-A- 1 949 095
- FR-A- 2 133 484
- FR-A- 2 429 797
- GB-A- 1 317 373
- GB-A- 1 398 026
- Z. CHEM. , Bd. 10, Nr. 8, 1970 Seiten 299-300, SCHWARZ S ET AL 'Steroids. XI. 17.alpha.-Ethynylestradiol sulfamates'
- PHARMAZIE, Bd. 30, Nr. 1, 1975 BERLIN DE, Seiten 17-21, SCHWARZ S ET AL 'Steroids. 15. Sulfonyloxy derivatives of estrogens'
- PHARMAZIE, Bd. 30, Nr. 1, 1975 BERLIN DE, Seiten 52-53, STOELZNER W ET AL 'Dissociation of the antigonadotrophic and the contraceptive activities of certain estratriene derivatives'
- CHEMICAL ABSTRACTS, vol. 091, no. 25, 17.Dezember 1979 Columbus, Ohio, US; abstract no. 204845, SHU H D ET AL 'Structure-activity relationships of estradiol derivatives' Seite 97; Spalte 2; & YAO HSUEH HSUEH PAO , Bd. 14, Nr. 6, 1979 1ST MED. COLL. SHANGHAI;DEP. PHARMACOL.; SHANGHAI; PEOP. R. CHINA, Seiten 343-348,
- JOURNAL OF MEDICINAL CHEMISTRY, Bd. 37, Nr. 2, 21.Januar 1994 WASHINGTON US, Seiten 219-221, HOWARTH N M ET AL 'Estrone sulfamates: potent inhibitors of estrone sulfatase with therapeutic potential'
- HELVETICA CHIMICA ACTA, Bd. 50, Nr. 1, 1967 BASEL CH, Seiten 281-288, J. KALVODA ET AL '7-alpha-Methylöstrogene'
- STEROIDS, Bd. 45, Nr. 3-4, 1985 SAN FRANCISCO US, Seiten 325-340, N. UBEROI ET AL 'Structure Activity Reltionships of some Unique Estrogens Related to Estradiol Are Predicted by Fit into DNA'
- JOURNAL OF MEDICINAL CHEMISTRY, Bd. 32, Nr. 7, 1989 WASHINGTON US, Seiten 1642-1652, R. PETERS ET AL '17-Desoxy Estrogen Analogues'
- STEROIDS: STRUCTURE, FUNCTION, AND REGULATION, Bd. 56, Nr. 4, 1991 MA US, Seiten 201-210, B. BHAVNANI ET AL 'Interaction of Ring-B Unsaturated Estrogens with Estrogen Receptors of Human Endometrium and Rat Uterus'
- STEROIDS, Bd. 36, Nr. 1, Juli 1980 SAN FRANCISCO US, Seiten 1-11, G. MERRIAM ET AL 'Comparative Properties of The Catechol Estrogens, I: Methylation by Catechol-O-Methyltransferase and Binding to Cytosol Estrogen Receptors'

## Beschreibung

Aufgabe der vorliegende Erfindung betrifft Estra-1,3,5 (10)-trien-Derivate, die am C-Atom 3 eine R-SO₂-O-Gruppe tragen, zur Herstellung von Arzneimitteln, die zur oralen Applikation geeignet sind, zur hormonalen Kontrazeption, zur Hormonsubstitutions-therapie und zur hormonden Behandlung von Prostakarzinomen.

Estrogene spielen in der hormonalen Kontrazeption und in der klimakterischen Hormon-Replacement-Therapie (HRT) sowie bei der Behandlung gynäkologischer (z. B. Mammacarcinom) und andrologischer (z. B. Prostatacarcinom) Krankheitsbilder eine wesentliche Rolle.

In der HRT und für die Kontrazeption werden Estrogene ganz überwiegend in Kombination mit einem Gestagen eingesetzt, z. B. Levonorgestrel, Desogestrel, Gestoden, Drospirorenon, Norethisteron, Cyproteronazetat, Chlormadinoazetat, Dienogest.

Im Falle der Kontrazeption werden Estrogene einmal dazu benötigt, um Follikelreifung und Ovulation sicher zu unterdrücken, andererseits substituieren sie dann die weitgehend unterdrückte endogene, ovarielle Sekretion von Estradiol. Diese Substitution ist wesentlich für die Erhaltung eines artifiziellen Menstruationzyklus und anderer Funktionen der Sexualorgane, die mit einem Gestagen allein nicht befriedigend gelingt.

Daneben haben endogene Estrogene wichtige zentralnervöse und metabolische Funktionen im weiblichen Organismus.

Normale Estrogenspiegel tragen zum Wohlbefinden entscheidend bei (L. Zichella; Clinical Management of the Menopausal Woman; Int. J. of Fertil. and Menop. Studies, 38, Suppl. 1 (1993), 15-22). Ihre Anwesenheit wirkt dem Entstehen von Herz-Kreislauf-Erkrankungen über verschiedene Mechanismen entgegen, nämlich durch Erzeugung von "günstigen" Lipoproteinmustern im Blut (G. Samsioe; Hormone Replacement Therapy and Cardiovascular Disease; Int. J. of Fertil. and Menop. Studies, 38, Suppl. 1 (1993), 23-29), Hemmung der Lipideinlagerung in der Gefäßwand (T. B. Clarkson; Experimental Effects of Progesterone versus Progestins on Arterial Wall; Gynecol. Endocrinol., 6: Suppl. 1 (1992), 15), Senkung des Blutdrucks durch günstige Beeinflussung des Gefäßtonus (R. A. Lobo; Estrogen and Cardiovascular Disease; Ann. New York Acad. Sciences, 592 (1990), 286-294), Reduktion des Perfusionswiderstandes in wichtigen Gefäßgebieten, Dämpfung kontraktiler Reize am Gefäßmuskel (C. Jiang et al.; Acute effect of 17β-estradiol on rabbit coronary artery contractile responses to endothelin-1; Am. J. Physiol., 263 (1992), H271-H275). Unter der Wirkung von Estrogenen setzen die Gefäßinnenwände Faktoren (Prostacyclin) frei, die der Entstehung von Blutgerinnseln entgegen wirken.

Estrogene sind bei der Frau zur Erhaltung der Knochenstruktur unerläßlich. Ihr Verlust kann die Entwicklung eines Knochenabbaus (Osteoporose) bewirken (C. Christiansen; Prevention and Treatment of Osteoporosis with Hormone Replacement Therapy; Int. J. of Fertil. and Menop. Studies, 38, Suppl. 1 (1993), 45-54). Die letztgenannten "zentralnervösen" und "metabolischen" Effekte der Estrogene sind ein wesentlicher Gesichtspunkt der HRT.

Bei allen positiven Aspekten der Estrogentherapie gibt es ungelöste Probleme, welche die therapeutische Anwendung von Estrogenen einschränken oder unerwünschte Wirkungen beinhalten.

Natürliche Estrogene (Estradiol, Estron, Estronsulfat, Ester von Estradiol, Estriol) werden bei oraler Anwendung nur zum geringsten Teil bioverfügbar (K. B. Lokind et al.; Oral bioavailability of 17β-estradiol and various ester prodrugs in the rat; Int. J. Pharmaceutics, 76 (1991), 177-182). Dieser Anteil ist individuell so variabel, daß generelle Dosisempfehlungen nicht möglich sind. Eine Verwendung natürlicher Estrogene (Estradiol) für hormonale Kontrazeption wurde wegen dieser pharmakokinetischen Gegebenheiten negativ beurteilt (W. Kuhnz et al.; Pharmacokinetics of Estradiol, Free and Total Estrone, in Young Women Following Single Intravenous and Oral Administration of 17β-Estradiol; Arzneimittel-Forschung/Drug Res., 43(II), 9, (1993), 966-973). Problematisch ist auch die rasche Eliminierung der Substanzen aus dem Blut. Die Estrogensubstitution in der HRT muß sehr oft individuell angepaßt werden. Die Entwicklung von Estradiolprodrugs mit dem Ziel, die orale Bioverfügbarkeit zu verbessern, führte zu negativen Ergebnissen (K. B. Lokind et al.; siehe oben).

Synthetische Estrogene weisen gleichfalls erhebliche Nachteile auf. Das wichtigste synthetisch abgewandelte estrogene Steroid ist das Ethinylestradiol (EE). Dieses Estrogen ist beherrschend in der oralen hormonalen Kontrazeption. Neben EE wird in wenigen Fällen das Mestranol eingesetzt, welches ein "Prodrug" ist und im Organismus zu EE verstoffwechselt wird (J. W. Goldzieher; Selected aspects of the pharmacokinetics and metabolism of ethinyl estrogens and their clinical implications; Am. J. Obstet. Gynecol., 163 (1990), 318-322). EE ist bei oraler Applikation (Mensch) viel besser bioverfügbar als die o. g. natürlichen Estrogene, allerdings variiert die orale Bioverfügbarkeit individuell außerordentlich stark. Goldzieher betonte unter pharmakodynamischen Gesichtspunkten die negative Bedeutung der Variation der Fläche unter der Kurve (AUC, area under the curve), der Halbwertszeit und der Zeit bis zu maximalen Blutspiegeln. Die höchste in dieser Studie gefundene AUC von 0-24 Stunden nach der Applikation war 2121 pg x h/ml. Der niedrigste AUC war 284 pg x h/ml. Eine ähnliche Streubreite der AUC um den Faktor 6 bis 7 ist auch in der Arbeit von Hümpel et al. beschrieben (M. Hümpel et al.; Comparison of Serum Ethinyl Estradiol, Sex-Hormone-Binding Globulin, Corticoid-Binding Globulin and Cortisol Levels in Women Using Two Low-Dose Combined Oral Contraceptives; Horm. Res., 33 (1990), 35-39).

Bei oraler Anwendung gelangen Wirkstoffe nach Resorption aus dem Darmlumen über die Leber in den Organismus. Für estrogene Wirkstoffe ist diese Tatsache von besonderer Bedeutung, da die Leber ein Erfolgsorgan für Estrogene ist und deren orale Gabe zu starken Estrogeneffekten in der Leber führt. Zu den Sekretionsaktivitäten, die in der menschlichen Leber durch Estrogene reguliert werden, gehören u. a. die Synthesen der Transportproteine CBG, SHBG, TBG, das Angiotensinogen, verschiedene Faktoren, die in der Physiologie der Blutgerinnung eine wichtige Rolle spielen und Lipoproteine.

Werden dem weiblichen Organismus natürliche Estrogene unter Umgehung der Leberpassage zugeführt, z. B. durch transdermale Applikation, so bleiben die genannten Leberfunktionen praktisch unverändert (U. Larsson-Cohn et al.; Some biochemical consequences of post-menopausal hormone replacement treatment; in: The Controversial Climacteric, Ed: P. A. van Keep et al.; MTP Press Ltd. (1982)). Therapeutisch äquivalente Dosen natürlicher Estrogene führen bei oraler Applikation zu deutlichen Reaktionen hepatischer Parameter: Anstieg von SHBG, CBG, Angiotensinogen, HDL (high density lipoproteins) (J. C. Stevenson et al.; Oral Versus Transdermal Hormone Replacement Therapy; Int. J. of Fertil. and Menop. Studies, 38, Suppl. 1 (1993), 30-35). Deutlich stärker ausgeprägt als bei natürlichen Estrogenen sind entsprechende hepatische Estrogeneffekte bei equinen Estrogenmischungen (sog. konjugierte Estrogene)(C. A. Mashchak et al.; Comparison of pharmacodynamic properties of various estrogen formulations; Am. J. Obstet. Gynecol., 144 (1982) 511-518). Noch stärkere hepatische Estrogenität besitzen das Ethinyl-Estradiol und das DES. Bezogen auf antigonadotrope Eigenschaften ist das EE in der Leber ca. 8-10 mal stärker estrogen wirksam als oral verabreichte natürliche Estrogene. Es liegt also eine sehr ungünstige Dissoziation von Eigenschaften vor (B. von Schoultz et al.; Estrogen Therapy and Liver Function - Metabolic Effects of Oral and Parenteral Administration; The Prostate, 14 (1989), 389-395).

Folgende Beobachtung zeigt, daß unerwünschte hepatische Estrogeneffekte nicht durch Dosisreduktion von EE in Kontrazeptiva vermeidbar sind. Die Reduktion von 30 µg auf 20 µg EE jeweils in Kombination mit 150 µg des gleichen Gestagens ergab nach 3 Monaten keine Reduktion der erheblich gestiegenen Angiotensinspiegel und allenfalls marginal reduzierte Werte nach 6 Monaten (A. Basdevant et al.; Hemostatic and metabolic effects of lowering the ethinylestradiol dose from 30 mcg to 20 mcg in oral contraceptives containing desogestrel; Contraception, 48 (1993), 193-204).

Im Falle der Estrogentherapie mit hoch dosierten Estrogenen bei Männern, die an Prostatacarcinom erkrankt sind, sind thromboembolische Komplikationen mit tödlichem Ausgang eine bekannte Komplikation (B. von Schoultz et al.; siehe oben).

In abgeschwächter Form bestimmt das Nebenwirkungspotential des EE in der Leber die Strategie der oralen hormonalen Kontrazeption.

Im Hinblick auf erwünschte kontrazeptive Effekte sowie die Erhaltung des monatlichen Menstruationsgeschehens einerseits und die Beachtung eines erheblichen Potentials von Nebenwirkungen andererseits, ist die schwere Steuerbarkeit der erwünschten Blutspiegel von EE ein großes Problem im Sinne einer Gratwanderung. Möglicherweise kann ein erheblicher Prozentsatz von Frauen orale Kontrazeptiva nicht anwenden, weil entweder Blutungsanomalien oder estrogenbedingte Nebenwirkungen die Akzeptanzgrenze überschreiten.

Unter hormonalen Kontrazeptiva steigt das Risiko, bestimmte kardiovaskuläre Erkrankungen zu erleiden und zu sterben, deutlich an (V. Wynn; Oral contraceptives and coronary disease; J. Reprod. Med., 36, Suppl. 3, (1991), 219-225). Da entsprechende Risiken altersabhängig sind (J. I. Mann; Oral contraceptives and myocardial infarction in young women; Pharmacol. steroid. Contracept. Drugs, Editors S. Garrattini und H. W . Berendes, Raven Press, New York, (1977), 289-296), haben verschiedene Gesundheitsbehörden davor gewarnt, hormonale Kontrazeptiva bei Frauen einzusetzen, die älter sind als 35 Jahre. Eklatante kardiovaskuläre Risiken bestehen bei Raucherinnen oberhalb von 35 Jahren, die hormonale Kontrazeptiva anwenden (F. A. Leidenberger; Klinische Endokrinologie für Frauenärzte, 382-383; J. I. Mann, siehe oben). Im Vergleich zu Kontrollpopulationen ist das Risiko tödlicher Kreislauferkrankungen bei Anwenderinnen oraler Kontrazeptiva um den Faktor 5 - 6 erhöht F.A. (Leidenberger, siehe oben). Diese Daten belegen, daß bei erheblichen Untergruppen geschlechtsreifer Frauen, herkömmliche hormonale Kontrazeptiva nicht oder nur mit unvertretbar hohem Risiko angewandt werden können.

Nach dem Stand der Wissenschaft ist die geschilderte Problematik dem Estrogenanteil in hormonalen Kontrazeptiva, nicht dem Gestagenanteil zuzuordnen (Skouby et al.; J. Obstet. Gynekol.; (1990), 1535-1537). Ein "Consensmeeting" kam zu der Feststellung, daß das Risiko von tödlchen Myokardinfarkten unabhängig von der Dauer der Anwendung besteht. Diese Feststellung belegt, daß die zum Tode führende Gerinnselbildung nicht etwa durch chronische Schädigungen der Arterienwände im Herzen erfolgt (Arteriosklerose), sondern durch akute Einflüsse auf Haemostasefunktionen in der Leber (R. A. Lobo, siehe oben). Die Reduktion von Estrogenwirkungen in der Leber erscheint daher geeignet, die geschilderten Risiken der hormonalen Kontrazeption und die geschilderten Anwendungsbeschränkungen zu eliminieren. Für natürliche Estrogene, d. h. Estrogene mit im Vergleich zu EE geringerer hepatischer Estrogenität, werden die für EE beschriebenen Risiken ausdrücklich ausgeschlossen (R. A. Lobo, siehe oben).

Die HRT mit natürlichen Hormonen erfordert mit der heutigen Technologie durchweg individuelle Dosisanpassungen. Entsprechende Behandlungen sind mit großen Unsicherheiten behaftet und beinhalten konkret die Gefahr von Über- und Unterdosierung.

Erfindungsgemäß verwendeten Verbindungen, die von der Breite der Formel I gedeckt sind, sind beispielsweise
17β-Hydroxy-estra-1,3,5(10)-trien-3-yl amidosulfonat,
17β-Hydroxy-estra-1,3,5(10)-trien-3-yl N,N-diethylamidosulfonat,
17β-Hydroxy-estra-1,3,5(10)-trien-3-yl N,N-dimethylamidosulfonat,
17β-Hydroxy-estra-1,3,5(10),7-tetraen-3-yl N,N-diethylamidosulfonat,
17β-Hydroxy-estra-1,3,5(10),6,8-pentaen-3-yl N,N-dimethylamidosulfonat,
17-Oxo-estra-1,3,5(10)-trien-3-yl amidosulfonat,
17-Oxo-estra-1,3,5(10)-trien-3-yl N,N-dimethylamidosulfonat,
17-Oxo-estra-1,3,5(10)-trien-3-yl N,N-diethylamidosulfonat,
16α,17β-Dihydroxy-estra-1,3,5(10)-trien-3-yl amidosulfonat,
16α,17β-Dihydroxy-estra-1,3,5(10)-trien-3-yl N,N-dimethylamidosulfonat,
16α,17β-Dihydroxy-estra-1,3,5(10)-trien-3-yl N,N-diethylamidosulfonat,
2,17β-Dihydroxy-estra-1,3,5(10)-trien-3-yl N,N-dimethylamidosulfonat,
2-Methoxy-17β-hydroxy-estra-1,3,5(10)-trien-3-yl N,N-diethylamidosulfonat,
17β-Hydroxy-14α,15α-methylen-estra-1,3,5(10)-trien-3-yl N,N-dimethylamidosulfonat,
17β-Hydroxy-14α,15α-methylen-estra-1,3,5(10)-trien-3-yl N,N-diethylamidosulfonat,
17β-Hydroxy-14α,15α-methylen-estra-1,3,5(10)-trien-3-yl pyrrolidinosulfonat,
17β-Hydroxy-14α,15α-methylen-estra-1,3,5(10)-trien-3-yl morpholinosulfonat,
17β-Hydroxy-14α,15α-methylen-estra-1,3,5(10)-trien-3-yl N-methylamidosulfonat,
17β-Hydroxy-14α,15α-methylen-estra-1,3,5(10)-trien-3-yl amidosulfonat,
17β-Hydroxy-14α,15α-methylen-estra-1,3,5(10),7-tetraen-3-yl N,N-dimethylamidosulfonat,
17β-Hydroxy-14α,15α-methylen-estra-1,3,5(10),6,8-pentaen-3-yl N,N-diethylamidosulfonat,
17β-Hydroxy-14α,15α-methylen-estra-1,3,5(10),8-tetraen-3-yl N,N-dimethylamidosulfonat,
11β-Chlormethyl-17β-hydroxy-estra-1,3,5(10)-trien-3-yl N,N-dimethylamidosulfonat,
17β-Hydroxy-14α,17α-vinylen-estra-1,3,5(10)-trien-3-yl N,N-diethylamidosulfonat,
14α,17α-Ethylen-17β-hydroxy-estra-1,3,5(10)-trien-3-yl pyrrolidinosulfonat,
16α,17β-Diydroxy-14α,17α-ethylen-estra-1,3,5(10)-trien-3-yl N,N-diethylamidosulfonat,
17β-Hydroxy-7α-methyl-estra-1,3,5(10)-trien-3,11β-diyl 3-N,N-dimethylamidosulfonat-11-nitrat und

Die erfindungsgemäße verwendeten Estra-1,3,5(10)-trien-Derivate sind der allgemeinen Formel I 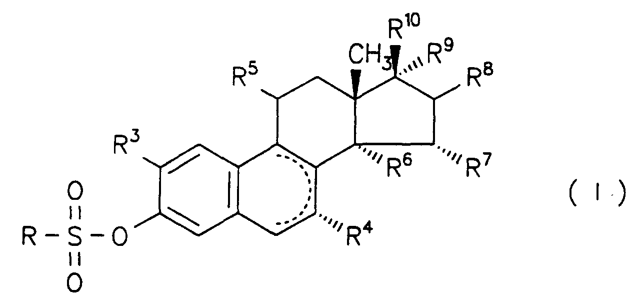 worin
R eine R¹R²N-Gruppe ist,
worin R¹ und R² unabhängig voneinander ein Wasserstoffatom, einen C₁-C₅-Alkylrest oder zusammen mit dem N-Atom einen Polymethyleniminorest mit 4 bis 6 C-Atomen oder einen Morpholinorest bedeuten,
R³ ein Wasserstoffatom, eine Hydroxygruppe oder eine Alkoxygruppe mit 1-5 C-Atomen bedeutet,
R⁴ ein Wasserstoffatom oder einen C₁-C₅-Alkylrest darstellt,
R⁵ ein Wasserstoffatom, eine Hydroxygruppe, eine veresterte Hydroxygruppe, eine Halogenalkyl- oder Alkoxygruppe mit 1-5 C-Atomen ist,
R⁶ und R⁷ Wasserstoffatome sind oder zusammen eine Methylengruppe darstellen,
R⁸, R⁹ und R¹⁰ unabhängig voneinander ein Wasserstoffatom oder eine Hydroxygruppe bedeuten
und der Ring B gegebenenfalls eine oder zwei Doppelbindungen enthält
oder
R⁹ und R¹⁰ zusammen ein Sauerstoffatom darstellen oder
R⁶ und R⁹ eine Vinylen- oder Ethylengruppe darstellen.

Besonders bevorzugte erfindungsgemäße verwendeten Estra-1,3,5(10)-trien-Derivate der allgemeinen Formel I, sind verbindungen wobei R⁵ und R⁶ Hydroxygruppen bedeuten.

Besonders bevorzugte erfindungsgemäße verwendeten Estra-1,3,5(10)-trien-Derivate der allgemeinen Formel I sind Verbindungen, wobei R³ und R⁴ zusammen eine Methylengruppe darstellen.

Insbesondere bevorzugt ist die Verwendung von:
17β-Hydroxy-estra-1,3,5(10)-trien-3-yl N,N-diethylamidosulfonat,
17β-Hydroxy-14α,15α-methylen-estra-1,3,5(10)-trien-3-yl N,N-diethylamidosulfonat,
16α,17β-Dihydroxy-estra-1,3,5(10)-trien-3-yl N,N-dimethylamidosulfonat,
Estra-1,3,5(10)-trien-17-on-3-yl N,N-diethylamidosulfonat,
17β-Hydroxy-14α,15α-methylen-estra-1,3,5(10)-trien-3-yl N,N-dimethylamidosulfonat,
   und
16α,17β-Dihydroxy-estra-1,3,5(10)-trien-3-yl N,N-diethylamidosulfonat.

Die erfindungsgemäßen verwendeten Wirkstoffe sind Estra-1,3,5(10)-trien-Derivate. Sie werden in an sich bekannter Weise hergestellt, in dem man ein Estra-1,3,5(10)-trien-Derivat mit einem entsprechend substituierten Amidosulfonylchlorid unter Veresterung der 3-OH-Gruppe des Estra-1,3,5(10)-trien-Derivats umsetzt.

Die Umsetzung erfolgt üblicherweise in einem 2-Phasen-System in Gegenwart eines quartären Ammoniumsalzes als Phasen-Transfer-Katalysator. Die Umsetzung erfolgt bei Temperaturen zwischen Raumtemperatur und 100°C. Als Lösungsmittel werden übliche 2-Pasen-Systeme, wie Chloroform-Wasser, Dichlormethan-Wasser, Toluol-Wasser u.a. verwendet.

Die in den erfindungsgemäßen Zusammensetzungen enthaltenen Verbindungen sind zum Teil bekannt.

So beschreibt WO93/0506 die Verwendung von Estron-3-Sulfamaten als Steroidsulfatase-Inhibitoren.

In der DD 207 447 wird die Verwendung von N,N-Dialkylsulfamat-Derivaten des Ethinylestradiols als Mittel zur Bekämpfung von schädlichen Nagetieren beschrieben.

Die DE-OS 2426779, DE-OS 2426778 und DE-OS 2426777 beschreiben ferner 3-Sulfamat-Derivate von Estratrienen, welche in der 1-Position eine weitere Hydroxylgruppe tragen.

Darüber hinaus beschreiben u. a. DD 77 709 und DD 114 806 die Darstellung der in den erfindungsgemäßen pharmazeutischen Zusammensetzungen enthaltenen Wirkstoffe.

Bisher sind aber keine pharmazeutischen Zusammensetzungen bekannt, welche als Wirkstoff die beanspruchten 3-Amidosulfonate von Estra-1,3,5(10)-trien-Derivaten enthalten und zur Kontrazeption, zur HRT und zur Carcinomtherapie eingesetzt werden können.

Der Gegenstand der vorliegenden Erfindung ist die Verwendung von der Verbindungen der Formel (I),
zur Herstellung von Arzneimitteln, die zur oralen Applikation geeignet sind, zur hormonalen Kontrazeption, klimakterischen Hormonsubstitutions-Therapie und zur Behandlung gynäkologischer und andrologischer Krankheitsbilder wie Mammacarinomen und Prostatacarcinomen.

Erfindungsgemäß werden beispielsweise
17β-Hydroxy-estra-1,3,5(10)-trien-3-yl amidosulfonat,
17β-Hydroxy-estra-1,3,5(10)-trien-3-yl N,N-diethylamidosulfonat,
17β-Hydroxy-estra-1,3,5(10)-trien-3-yl N,N-dimethylamidosulfonat,
17β-Hydroxy-estra-1,3,5(10),7-tetraen-3-yl N,N-diethylamidosulfonat,
17β-Hydroxy-estra-1,3,5(10),6,8-pentaen-3-yl N,N-dimethylamidosulfonat,
17-Oxo-estra-1,3,5(10)-trien-3-yl amidosulfonat,
17-Oxo-estra-1,3,5(10)-trien-3-yl N,N-dimethylamidosulfonat,
17-Oxo-estra-1,3,5(10)-trien-3-yl N,N-diethylamidosulfonat,
16α,17β-Dihydroxy-estra-1,3,5(10)-trien-3-yl amidosulfonat,
16α,17β-Dihydroxy-estra-1,3,5(10)-trien-3-yl N,N-dimethylamidosulfonat,
16α,17β-Dihydroxy-estra-1,3,5(10)-trien-3-yl N,N-diethylamidosulfonat,
2,17β-Dihydroxy-estra-1,3,5(10)-trien-3-yl N,N-dimethylamidosulfonat,
2-Methoxy-17β-hydroxy-estra-1,3,5(10)-trien-3-yl N,N-diethylamidosulfonat,
17β-Hydroxy-14α,15α-methylen-estra-1,3,5(10)-trien-3-yl N,N-dimethylamidosulfonat,
17β-Hydroxy-14α,15α-methylen-estra-1,3,5(10)-trien-3-yl N,N-diethylamidosulfonat,
17β-Hydroxy-14α,15α-methylen-estra-1,3,5(10)-trien-3-yl pyrrolidinosulfonat,
17β-Hydroxy-14α,15α-methylen-estra-1,3,5(10)-trien-3-yl morpholinosulfonat,
17β-Hydroxy-14α,15α-methylen-estra-1,3,5(10)-trien-3-yl N-methylamidosulfonat,
17β-Hydroxy-14α,15α-methylen-estra-1,3,5(10)-trien-3-yl amidosulfonat,
17β-Hydroxy-14α,15α-methylen-estra-1,3,5(10),7-tetraen-3-yl N,N-dimethylamidosulfonat,
17β-Hydroxy-14α,15α-methylen-estra-1,3,5(10),6,8-pentaen-3-yl N,N-diethylamidosulfonat,
17β-Hydroxy-14α,15α-methylen-estra-1,3,5(10),8-tetraen-3-yl N,N-dimethylamidosulfonat,
11β-Chlormethyl-17β-hydroxy-estra-1,3,5(10)-trien-3-yl N,N-dimethylamidosulfonat,
17β-Hydroxy-14α,17α-vinylen-estra-1,3,5(10)-trien-3-yl N,N-diethylamidosulfonat,
14α,17α-Ethylen-17β-hydroxy-estra-1,3,5(10)-trien-3-yl pyrrolidinosulfonat,
16α,17β-Diydroxy-14α,17α-ethylen-estra-1,3,5(10)-trien-3-yl N,N-diethylamidosulfonat,
17β-Hydroxy-7α-methyl-estra-1,3,5(10)-trien-3,11β-diyl 3-N,N-dimethylamidosulfonat-11-nitrat verwendet.

Die erfindungsgemäßen verwendeten Verbindungen werden in pharmazeutischen Zusammensetzungen verwendet, worin gegebenenfalls zusätzlich eines oder mehrere der bereits erwähnten Gestagene, wie z. B. Levonorgestrel, Desogestrel, Gestoden, Drospirorenon, Norethisteron, Cyproteronazetat, Chlormadinoazetat, Dienogest vorliegen.

Ferner können die erfindingsgemäßen verwendeten Verbindungen in Form von Mehrstufen- oder Kombinationspräparaten vorliegen.

Einderartiges Kombinationspräparat zur Kontrazeption besteht beispielsweise aus einer ersten Stufe, die eine Kombination mehrerer Komponenten, nämlich einem biogenen Estrogen, einem synthetischen Estrogen, einem Gestagen und/oder einem Estra-1,3,5(10)-trien-Derivata der Formel (I), enthält, sowie gegebenenfalls einer oder mehreren weiteren Stufen, die aus einem pharmazeutisch unbedenklichen Placebo oder einem biogenen oder synthetischen Gestagen oder einem biogenen oder synthetischen Estrogen oder einem Estra-1,3,5(10)-trien-Derivat der Formel (I), oder aus einer Kombination aus mehreren Komponenten, nämlich einem biogenen Estrogen, einem synthetischen Estrogen, einem Gestagen, einem Estra-1,3,5(10)-trien-Derivat der Formel (I), oder einer Kombination aus synthetischen Estrogenen oder einem Estra-1,3,5(10)-trien-Derivat der Formel (I), und einem Gestagen bestehen.

Das biogene Estrogen weist beispielsweise einen Bestandteil aus der Gruppe Estradiol, Estron, Estran, Estriol und anderen biogenen Estrogenen oder mindestens eine Verbindung, die einen der vorgenannten Estrogenbestandteile nach Einnahme schnell abspaltet, auf.

Das synthetische Estrogen weist mindestens einen Bestandteil aus der Gruppe Ethinylestradiol, Mestranol und anderen synthetischen Estrogenen oder mindestens eine Verbindung, die einen der vorgenannten Estrogenbestandteile nach Einnahme schnell abspaltet, auf.

Das Gestagen weist erfindungsgemäß mindestens einen Bestandteil aus der Gruppe Levonorgestrel, Desogestrel, Dienogest, Progesteron, Norethisteronacetat, Chlormadinonacetat. Gestoden, Cyproteronacetat und anderen natürlichen und/oder synthetischen Gestagenen oder mindestens eine Verbindung, die einen der vorgenannten Gestagenbestandteile nach Einnahme schnell abspaltet, auf.

Die erfindungsgemäß verwendeten Verbindungen können als pharmazeutische Zusammensetzungen in Form von Tabletten, Tabletten mit kontrollierter Freisetzung, Dragees, Pillen, Kapseln, Filmtabletten und Filmtabletten mit kontrollierter Freisetzung verwendet werden.

Die erfindungsgemäß hergestellten Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver oder Depotformen.

Entsprechende Tabletten können beispielsweise durch Mischen des Wirkstoffes mit bekannten Hilfsstoffen, beispielsweise inerten Verdünnungsmitteln wie Dextrose, Zukker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Wegen der schwerwiegenden Nachteile der herkömmlichen auf medizinischem Gebiet eingesetzten Estrogenderivate besteht jedoch ein dringendes Bedürfnis nach geeigneten pharmazeutischen Zusammensetzungen, welche die oben genannten Nachteile nicht aufweisen.

Obwohl die in den erfindungsgemäßen pharmazeutischen Zusammensetzungen enthaltenen Wirkstoffe zum Teil schon lange bekannt sind und pharmakologisch gut untersucht wurden, sind deren günstige Eigenschaften im Hinblick auf hepatische Funktionen bisher nicht beschrieben worden.

Überraschenderweise wurde gefunden, daß die erfindungsgemäß verwendeten Wirkstoffe, welche EE hinsichtlich estrogener Wirksamkeit übertreffen, aber bei maximalen genitalen Estrogeneffekten im Uterus, hepatisch nicht stärker estrogen sind als das natürliche Estrogen Estradiol. Durch diese Konstellation werden mit den erfindungsgemäß verwendeten Wirkstoffen die therapeutischen Eigenschaften im Vergleich zu natürlichen und synthetischen Estrogenen entscheidend verbessert.

Kontrazeptiva, welche Estra-1,3,5(10)-trien-Derivate der Formel I, enthalten, sind geeignet, Anwendungsbeschränkungen für hormonale Kontrazeption völlig neu zu definieren, da sie weniger oder keine Wirkung auf das Haemostasesystem haben.

Kontrazeptiva, welche Estra-1,3,5(10)-trien-Derivate der Formel I, enthalten, können wegen dramatisch gesenkter Estrogeneffekte so hoch dosiert werden, daß auch eine bessere Zykluskontrolle als mit herkömmlichen EE-Kontrazeptiva möglich ist.

Die Anwendung von Ethinylestradiol (EE) für die Hormonsubstituions-Therapie wird wegen der Nebenwirkungsproblematik z. Z. strikt abgelehnt. Mit den erfindungsgemäß verwendeten Estra-1,3,5(10)-trien-Derivaten, welche am C-Atom 3 eine R-SO₂-O-Gruppierung tragen und worin R die oben angegebene Bedeutung hat, bestehen die für die nicht natürlichen (biogenen) Estrogene bestehenden Risiken nicht mehr. Im Vergleich zu den heute in der Hormonsubstituions-Therapie dominierenden natürlichen Estrogenen besthet der Vorteil einer weit überlegenen Steuerbarkeit, da die orale Bioverfügbarkeit definiert ist und nicht wie bei den biogenen Estrogenen individuell stark unterschiedlich ist.

Der Nachweis der hepatischen Estrogenität erfolgte an ovariektomierten Ratten. Die Versuchstiere, adulte weibliche Ratten (Züchter: HSD/WIN:WU), werden ovariektomiert (Tag -14). Zwei Wochen später beginnt die Behandlung durch 1 mal tägliche orale Applikation der jeweiligen Testsubstanzen. Die angegebenen Dosierungen beziehen sich bei Estern auf den Steroidanteil der Substanzen. Die Versuchsgruppen und die jeweiligen Tierzahlen sind in Tabelle 1 dargestellt. Die Versuche wurden in drei Gruppen A, B und C zu verschiedenen Zeitpunkten durchgeführt. Dadurch ergibt sich die jeweilige Abweichung in den Werten der Kontrollgruppen. Die Versuche der Versuchsreihen A, B und C sind somit unter Berücksichtigung der Werte der jeweiligen Kontrollgruppen miteinander vergleichbar.

**Tabelle 1:**

| Übersicht der Versuche, der geprüften Substanzen, der Tierzahlen und der Dosierungen | | | | | | | | | |
|---|---|---|---|---|---|---|---|---|---|
| | Versuch A | | | | | Versuch B | | Versuch C | |
| Substanzen | Dosierung (µg/Tier/Tag/p.o. d1-d7) | | | | | | | | |
| | 0 | 0,1 | 1 | 10 | 100 | 0 | 10 | 0 | 10 |
| Kontrolle | 16 | | | | | 14 | | 8 | |
| E2 (1) | | 6 | 6 | 6 | 6 | | 8 | | |
| EE (1) | | 6 | 6 | 6 | 6 | | 8 | | |
| J 824 (1) | | 6 | 6 | 6 | 6 | | 8 | | |
| J 271 (B 2) | | 6 | 6 | 6 | 6 | | 8 | | |
| J 981 (B 4) | | | | | | | 8 | | |
| J 272 (B 3) | | | | | | | 8 | | |
| J 665 (B 8) | | | | | | | 8 | | |
| J 982 (B 9) | | | | | | | 8 | | |
| J 983 (B 5) | | | | | | | 8 | | |
| J 893 (1) | | | | | | | 8 | | |
| E1 (1) | | | | | | | 8 | | |
| J 804 (B 7) | | | | | | | 8 | | |
| E3 (1) | | | | | | | | | 8 |
| J 984 (B 6)? | | | | | | | | | 7 |
| J 989 (B 6)? | | | | | | | | | 8 |
| (1) Vergleichssubstanzen | | | | | | | | | |
| (B X) Erfindungsgemäß verwendete Substanzen mit Nummer des Beispiels X | | | | | | | | | |
| E2 (Estradiol), EE (Ethinylestradiol), E1 (Estron) | | | | | | | | | |
| E3 (Estratriol) | | | | | | | | | |

Die Zuordnung der einzelnen Tiere zu ihren Gruppen erfolgte durch Randomisierung. Der Versuch wurde als Blockversuch durchgeführt. Die Tiere wurden vor Versuchsbeginn und bei Versuchsende gewogen.

Behandlungsbeginn ist definiert als Tag 1(=d1), Behandlungsende ist Tag 7 (=d7), am Tag 8 wurden die Tiere getötet, verschiedene Organe (Uteri, Nebennieren, Leber) entnommen, gewogen und für weitere Untersuchungen tiefgekühlt (-196°C) weitergegeben.

Blut wurde in Ethernarkose vor der Behandlung (d0) bzw. (d4) und (d8) aus dem retrobulbären Plexus entnommen. Im gewonnenen Serum wurden IGF₁, Angiotensin I, Cholesterin und HDL-Cholesterin bestimmt.

Bestimmungsmethoden:
IGF₁ - RIA Firma bioMérieux;
Angiotensin - Modifizierter RIA für Reninaktivität, Firma Sorin;
Cholesterin/HDL - enzymatische Tests, photometrische Bestimmung, Reagentien Firma Dr. Bruno Lange GmbH.

Die Ergebnisse der Untersuchungen sind in den folgenden Tabellen 2 bis 7 b dargestellt:

### Effekte auf Uteruswachstum (vgl. Tabelle 2)

Die Wirkung der Verbindung nach Beispiel 2 hat bereits bei einer Dosis von 0,01 mg/Tier/Tag oral ein Plateau erreicht. Es besteht eine deutliche Überlegenheit vs EE und eine starke Überlegenheit gegenüber Estradiol.

**Tabelle 2:**

| Orale Estrogeneffekte: Beeinflussung der Sexualfunktion Uterusgewichte (mg, Mittelwert ± Standardabweichung) in Abhängigkeit von der Dosierung | | | | | | | | | |
|---|---|---|---|---|---|---|---|---|---|
| | Versuch A | | | | | (Versuch B | | Versuch C | |
| Substanzen | Dosierung (µg/Tier/Tag/p.o. d1-d7) | | | | | | | | |
| | 0 | 0,1 | 1 | 10 | 100 | 0 | 10 | 0 | 10 |
| Kontrolle | 149,07 | | | | | 164,08 | | 185,4± | |
| | ±17,52 | | | | | ±13,5 | | 27,0 | |
| E2 | | 123,3±21,2 | 128,9±9,8 | 130,7±11,7 | 285,4±68,2 | | 182,3±16,78 | | |
| EE | | 152,2±20,8 | 195,8±31,1 | 282,4±42,2 | 441,2±46,3 | | 352,59±37,54 | | |
| J 824 | | 147,9±14,4 | 220,1±48,4 | 435,3±76,9 | 559,1±83,6 | | 537,5±102,7 | | |
| J 271 | | 144,2±19,6 | 289,9±22,3 | 605,1±118,5 | 563,6±44,3 | | 540,8±56,1 | | |
| J 981 | | | | | | | 225,34±37,3 | | |
| J 272 | | | | | | | 620,7±104,3 | | |
| J 665 | | | | | | | 639,48±82,83 | | |
| J 982 | | | | | | | 193,4±20,1 | | |
| J 983 | | | | | | | 348,6±78,8 | | |
| J 893 | | | | | | | 524,5±65,8 | | |
| | | | | | | | 170,63±22,8 | | |
| J 804 | | | | | | | 166,0±22,5 | | |
| E3 | | | | | | | | | 301,94±58,5 |
| J 984 | | | | | | | | | 245,63±57,0 |
| J 989 | | | | | | | | | 182,79±20,5 |

**Tabelle 3:**

| Orale Estrogeneffekte: Beeinflussung der Nebennierenfunktion Nebennierengewichte (mg, Mittelwert ± Standardabweichung) in Abhängigkeit von der Dosierung | | | | | | | | | |
|---|---|---|---|---|---|---|---|---|---|
| | Versuch A | | | | | Versuch B | | Versuch C | |
| Substanzen | Dosierung (µg/Tier/Tag/p.o. d1-d7) | | | | | | | | |
| | 0 | 0,1 | 1 | 10 | 100 | 0 | 10 | 0 | 10 |
| Kontrolle | 59,99±8,8 | | | | | 53,0±3,9 | | 53,1±4,2 | |
| E2 | | 49,3±4,3 | 57,9±6,52 | 61,6±7,8 | 54,4±9,1 | | 54,4±2,7 | | |
| EE | | 53,7±8,6 | 58.9±8,0 | 59,0±5,8 | 80,8±7,3 | | 56,6±6,6 | | |
| J 824 | | 54,5±8,4 | 56,7±6,7 | 74,8±12,2 | 76,1±9,7 | | 63,7 7,3 | | |
| J 271 | | 57,1±7,6 | 57,4±7,9 | 63,1±6,3 | 66,5±8,9 | | 57,9±6,0 | | |
| J 981 | | | | | | | 50,5±3,9 | | |
| J 272 | | | | | | | 57,5±8,2 | | |
| J 665 | | | | | | | 58,0±7,8 | | |
| J 982 | | | | | | | 55,3±5,8 | | |
| J 983 | | | | | | | 53.9±4,0 | | |
| J 893 | | | | | | | 53,5±6,3 | | |
| E1 | | | | | | | 49,8±5,0 | | |
| J 804 | | | | | | | 53,9±8,1 | | |
| E3 | | | | | | | | | 52,2±8,4 |
| | | | | | | | | | 59,3±7,6 |
| J989 | | | | | | | | | 52,8±6,4 |

### Effekte auf Nebennierengewicht (vgl. Tabelle 3)

Dosisabhängig sind Gewichtssteigerungen der Nebennieren zu beobachten. Ausnahme: Estradiol, deutlich geringere Steigerung vs EE.

### Effekte auf IGF₁ (vgl. Tabelle 4)

Bei einer Dosis von 100 µg/Tier reduzieren alle getesteten Substanzen im Verlauf des Versuchs das IGF₁. Ausnahme: Estradiol

**Tabelle 4:**

| Orale Estrogenbehandlung: IGF₁ (=Somatomedin C) als Ausdruck der hypophysären Sekretion von Wachstumshormonen nach R. Krattenmacher et al. (J. Steroid. Biochem. Molec. Biol.; 48 (23), 207-214 (1994)) | | | |
|---|---|---|---|
| **Substanzen** | **Dosierung (100 µg/Tier/Tag p.o. d1-d7)** | | |
| **Versuch A** | **d 0** | **d 4** | **d 8** |
| **Kontrolle** | **825,7 ± 56,5** | **811,4 ± 77,3** | **774,8 ± 82,5** |
| **E2** | **717,4 ± 80,8** | **623,6 ± 100,0** | **601,1 ± 108,8** |
| **EE** | **704,6 ± 70,4** | **547,0 ± 88,7** | **393,6 ± 71,1** |
| **J 271** | **826,4 ± 96,7** | **593,7 ± 57,0** | **351,5 ± 32,6** |
| **J 824** | **733,0 ± 136,6** | **504,3 ± 131,8** | **341,4 ± 100,0** |

### Angiotensin I (AI) (vgl. Tabellen 5a und 5b)

Verläufe der AI-Werte unter verschiedenen Dosierungen (1,0 bzw. 10,0 bzw. 100,0 µg/Tier/Tag, ausgewählte Substanzen) :

Bei der geringsten Dosis sind nur unter J 824 erhöhte AI-Werte erkennbar. Estradiol hat bei keiner Dosis erkennbare Effekte bei Versuchsende. EE und J 824 führen bei Dosierungen von 10 bzw. 100 µg zu starken Erhöhungen des AI, die bereits am Tag 4 der Behandlung erkennbar sind J271 hat bei einer Dosis von 10 µg/Tag noch keine deutlichen Effekte auf AI. Bei einer Dosis von 100 µg/Tag kommt es auch unter dieser Substanz zu Anstiegen von AI, diese sind aber deutlich geringer als unter EE oder J 824 bei dieser Dosis.

**Tabelle 5 a:**

| Effekte oraler Estrogenbehandlung auf hepatische Parameter Angiotensin I - Dosis-Wirkungs-Beziehung am Tag 4 | | | | |
|---|---|---|---|---|
| **Substanzen** | **Dosierung (µg/Tier/Tag p.o. d1-d7)** | | | |
| **Versuch A** | **0,1** | **1** | **10** | **100** |
| **Kontrolle** | **426,4± 20,6** | **424,9± 33,1** | **403,6± 24,4** | **406,1± 65,4** |
| **E2** | **376,2± 48,3** | **359,8± 53,4** | **412,8± 66,6** | **497,7± 69,0** |
| **EE** | **408,2± 31,2** | **402,2± 30,3** | **572,2± 35,0** | **930,1± 146,2** |
| **J 271** | **380,8± 35,8** | **383,8± 47,1** | **435,2± 53,8** | **734,0± 75,7** |
| **J 824** | **434,3± 65,5** | **355,2± 29,5** | **658,9± 18,8** | **1029,7±174,7** |

**Tabelle 5 b:**

| Effekte oraler Estrogenbehandlung auf hepatische Parameter Angiotensin I - Dosis-Wirkungs-Beziehung am Tag 8 | | | | |
|---|---|---|---|---|
| **Substanzen** | **Dosierung (µg/Tier/Tag p.o. d1-d7)** | | | |
| **Versuch A** | **0,1** | **1** | **10** | **100** |
| **Kontrolle** | **436,2± 11,9** | **447,1± 30,0** | **346,6± 50,5** | **353,1± 42,1** |
| **E2** | **449,5± 55,1** | **392,9± 32,0** | **335,5± 24,9** | **464,3± 16,7** |
| **EE** | **473,7± 19,7** | **492,6± 29,1** | **498,7± 26,1** | **833,1± 137,2** |
| **J 271** | **462,2± 71,3** | **425,9± 43,9** | **373,4± 42,3** | **668,2± 68,6** |
| **J 824** | **482,2± 39,8** | **606,7± 188,2** | **655,0± 64,7** | **958,3± 207,6** |

**Tabelle 6 a:**

| Effekte oraler Estrogenbehandlung auf hepatische Parameter Gesamtcholesterinspiegel (mg/dl, Mittelwert ± Standardabweichung) Dosis-Wirkungs-Beziehung am Tag 4 | | | | |
|---|---|---|---|---|
| **Substanzen** | **Dosierung (µg/Tier/Tag p.o. d1-d7)** | | | |
| **Versuch A** | **0,1** | **1** | **10** | **100** |
| **Kontrolle** | **113,4± 21,8** | **113,7± 12,5** | **94,9± 12,6** | **85,2± 13,3** |
| **E2** | **91,4± 10,6** | **94,2± 16,8** | **94,9± 10,2** | **74,3± 10,7** |
| **EE** | **81,0± 3,3** | **82,4± 15,8** | **30,3± 9,6** | **6,8± 3,2** |
| **J 271** | **96,0± 15,0** | **108,5± 10,3** | **92,4± 12,1** | **24,5± 14,4** |
| **J 824** | **94,2± 14,6** | **68,5± 7,1** | **16,6± 2,8** | **6,9± 7,5** |

**Tabelle 6 b:**

| Effekte oraler Estrogenbehandlung auf hepatische Parameter Gesamtcholesterinspiegel (mg/dl, Mittelwert ± Standardabweichung) Dosis-Wirkungs-Beziehung am Tag 8 | | | | |
|---|---|---|---|---|
| **Substanzen** | **Dosierung (µg/Tier/Tag p.o. d1-d7)** | | | |
| **Versuch A** | **0,1** | **1** | **10** | **100** |
| **Kontrolle** | **111,7± 20,9** | **113,6± 17,7** | **93,0± 13,2** | **93,4± 10,3** |
| **E2** | **92,7± 7,6** | **88,8± 7,5** | **98,5± 5,9** | **68,4± 12,7** |
| **EE** | **86,5± 9,6** | **80,2± 14,4** | **39,8± 10,2** | **5,5± 3,3** |
| **J 271** | **91,1± 18,3** | **101,4 ± 6,3** | **81,9 ± 12,6** | **18,6± 8,3** |
| **J 824** | **92,3 ± 12,6** | **70,0± 9,0** | **18,1± 3,3** | **8,4± 10,7** |

**Tabelle 7 a:**

| Effekte oraler Estrogenbehandlung auf hepatische Parameter HDL-Cholesterinspiegel (mg/dl, Mittelwert ± Standardabweichung) Dosis-Wirkungs-Beziehung am Tag 4 | | | | |
|---|---|---|---|---|
| **Substanzen** | **Dosierung (µg/Tier/Tag p.o. d1-d7)** | | | |
| **Versuch A** | **0,1** | **1** | **10** | **100** |
| **Kontrolle** | **61,7 ± 5,7** | **58,4 ± 7,0** | **60,5± 11,3** | **51,2± 4,9** |
| **E2** | **49,1 ± 7,8** | **50,9± 6,1** | **55,0± 6,9** | **43,7± 7,6** |
| **EE** | **49,6 ± 2,9** | **47,1 ± 8,5** | **19,5 ± 8,4** | **3,6 ± 1,3** |
| **J 271** | **57,6 ± 6,5** | **57,1± 7,1** | **54,6 ± 5,7** | **13,2 ± 14,0** |
| **J 824** | **52,5± 7,4** | **40,3± 8,0** | **7,1± 2,0** | **4,7± 5,4** |

**Tabelle 7 b:**

| Effekte oraler Estrogenbehandlung auf hepatische Parameter HDL-Cholesterinspiegel (mg/dl, Mittelwert ± Standardabweichung) Dosis-Wirkungs-Beziehung am Tag 8 | | | | |
|---|---|---|---|---|
| **Substanzen** | **Dosierung (µg/Tier/Tag p.o. d1-d7)** | | | |
| **Versuch A** | **0,1** | **1** | **10** | **100** |
| **Kontrolle** | **62,9± 13,2** | **60,2± 2,5** | **55,9± 10,2** | **58,8± 10,9** |
| **E2** | **51,0± 4,6** | **52,3± 4,1** | **51,5± 3,9** | **46,0± 9,2** |
| **EE** | **52,2± 4,1** | **49,5± 6,9** | **26,5± 7,8** | **2,4± 0,9** |
| **J 271** | **57,3± 6,0** | **59,6± 6,8** | **49,9± 6,0** | **10,7± 8,2** |
| **J 824** | **55,3± 8,8** | **48,2± 6,1** | **7,2± 2,4** | **5,0± 7,9** |

Dosis-Wirkungs-Beziehungen am Tag 4 und Tag 8 für Angiotensin I, Gesamtcholesterin und HDL-Cholesterin (vgl. Tabellen 5 a, 5 b, 6 a, 6 b, 7 a und 7 b):

### Angiotensin I (vgl. Tabellen 5 a und 5 b):

Estradiol hat allenfalls marginal stimulierende Effekte auf Angiotensin I-Spiegel. Die Werte unter der Verbindung nach Beispiel 2 liegen deutlich unter denen von EE.

Gesamtcholesterinspiegel (vgl. Tabellen 6 a und 6 b) sowie HDL-Cholesterinspiegel (vgl. Tabellen 7 a und 7 b): Bei der Ratte senken Estrogene die Blutcholesterinspiegel dosisabhängig sehr stark. Die beobachteten Trends sind unter der Behandlung für HDL-Cholesterol und Gesamtcholesterol gleichsinnig. Dosierungen von 10 und 100 µg führen bei den meisten geprüften Substanzen zu sehr niedrigen Cholesterolblutwerten. Verschiedene Substanzen führen bereits bei Dosen von 1 µg/Tier zu deutlichen Veränderungen. Lediglich Estradiol (alle Dosierungen) und die Verbindung nach Beispiel 2 bis inclusive 10 µg führen nicht zur Senkung der Cholesterinwerte. Auch die höchste Dosis der Verbindung nach Beispiel 2 führt noch zu erkennbar geringerem Abfall von Gesamtcholesterin und HDL-Cholesterol im Blut als EE.

Die folgenden Beispiele.erläutern die Erfindung:

### Beispiel 1:

Allgemeine Herstellungsvorschrift für die Darstellung von 3-Amidosulfonyloxy-Derivaten von Estra-1,3,5(10)-Derivaten der Formel I.

Zu einem Gemisch eines geeigneten organischen Lösungsmittels und Wasser gibt man unter heftigem Rühren das zu veresternde Estra-1,3,5(10)-Derivat, Alkali- oder Erdalkalihydroxid und quartäres Ammoniumsalz als Phasentransfer-Katalysator.
Man rührt so lange weiter, bis der analytische Nachweis (Dünnschicht-Chromatographie) einen vollständigen Verlauf der Veresterung anzeigt, wobei gegebenenfalls zwecks Verkürzung der Reaktionszeit bei Temperaturen von 50°C bis 100°C gearbeitet werden kann.
Danach werden die beiden Phasen getrennt. Die wäßrige Phase wird nachextrahiert und die vereinigten organischen Extrakte wäscht man nacheinander mit verdünnter Salzsäure, gesättigter Natriumhydrogencarbonatlösung und Wasser. Anschließend wird der Extrakt über wasserfreiem Natriumsulfat getrocknet und unter vermindertem Druck zur Trockne eingeengt. Der Rückstand wird aus einem geeigneten Lösungsmittel umkristallisiert.

### Beispiel 2 (= J 271): (Vergleichs beispiel).

### Herstellung von 17β-Hydroxy-19-nor-17a-pregna-1,3,5(10)-trien-20-in-3-yl N,N-diethylamidosulfonat

1 g 17α-Ethinylestradiol, 0,4 g Natriumhydroxid, 0,08 g Benzyltriethylammoniumchlorid und 1,16 g N,N-Diethylamidosulfonylchlorid werden wie in Beispiel 1 beschrieben in einem Gemisch aus 5 ml Dichlormethan und 2,5 ml Wasser zur Reaktion gebracht.
Nach Aufarbeitung und Umkristallisation des Rohproduktes aus Diisopropylether erhält man die Titelverbindung.
Fp.: 113-115 °C; [α]D: +3° (Chloroform, c=1)

### Beispiel 3 (= J 272): (Vergleichs beispiel).

### Herstellung von 17β-Hydroxy-19-nor-17α-pregna-1,3,5(10)-trien-20-in-3-yl pyrrolidinosulfonat

1 g 17α-Ethinylestradiol, 0,57 g Kaliumhydroxid, 0,08 g Benzyltriethylammoniumchlorid und 1,15 g Pyrrolidinosulfonylchlorid werden wie in Beispiel 1 beschrieben in einem Gemisch aus 5 ml Dichlormethan und 2,5 ml Wasser zur Reaktion gebracht.
Nach Aufarbeitung und Umkristallisation des Rohproduktes aus Diethylether erhält man die Titelverbindung.
Fp.: 121-122 °C; [α]D: +10° (Chloroform, c=1)

### Beispiel 4 (= J 981):

### Herstellung von 17β-Hydroxy-estra-1,3,5(10)-trien-3-yl N,N-diethylamidosulfonat

0,92 g Estradiol, 0,4 g Natriumhydroxid, 0,08 g Benzyltriethylammoniumchlorid und 1,16 g N,N-Diethylamidosulfonylchlorid werden wie in Beispiel 1 beschrieben in einem Gemisch aus 5 ml Dichlormethan und 2,5 ml Wasser zur Reaktion gebracht.
Nach Aufarbeitung und Umkristallisation des Rohproduktes aus Methanol erhält man die Titelverbindung.
Fp.: 175-176 °C; [α]D: +57° (Chloroform, c=1)

### Beispiel 5 (= J 983):

### Herstellung von 17β-Hydroxy-14α,15α-methylen-estra-1,3,5(10)-trien-3-yl N,N-diethylamidosulfonat

2 g 14α,15α-Methylen-estra-1,3,5(10)-trien-3,17β-diol werden mit 30 ml Toluol, 4 ml Wasser, 0,32 g Benzyltriethylammoniumchlorid, 2,94 ml N,N-Diethylamidosulfonylchlorid und 2,1 ml 40%iger Natronlauge suspendiert und unter Rühren zwei Stunden lang auf eine Innentemperatur von 80°C erwärmt.
Nach dem Abkühlen auf Raumtemperatur wird wie in Beispiel 1 angegeben aufgearbeitet. Das erhaltene Rohprodukt wird an Kieselgel (Korngröße 0,063 bis 0,2 mm) chromatographiert. Nach Elution mit Chloroform/Ethylacetat 9:1 und Umkristallisation aus Methanol erhält man die Titelverbindung.
Fp.: 68-73 °C; [a]D: +98° (Chloroform, c=1)

### Beispiel 6 (= J 989)

### Herstellung von 16α,17β-Dihydroxy-estra-1,3,5(10)-trien-3-yl N,N-dimethylamidosulfonat

Zu einer Lösung von 2 g Estriol in 800 ml Toluol bei einer Temperatur von 80°C gibt man unter Rühren 120 ml Wasser, 1,58 g Benzyltriethylammoniumchlorid, 7,44 ml N,N-Dimethylamidosulfonylchlorid und 4 ml 40%ige Natronlauge. Man erwärmt weiter auf 80°C. Während dieser Zeit wird der pH-Wert 10 der Reaktionslösung durch Zugabe von 40%iger Natronlauge aufrechterhalten. Nach vollständiger Umsetzung der Ausgangsverbindungen kühlt man auf Raumtemperatur ab und arbeitet wie in Beispiel 1 angegeben auf.
Der erhaltene Rückstand wird aus Aceton/n-Hexan umkristallisiert und ergibt die Titelverbindung.
Fp.: 180-181 °C; [α]D: +48° (Chloroform, c=1)

### Beispiel 7 (= J 804):

### Herstellung von Estra-1,3,5(10)-trien-17-on-3-yl N,N-diethylamidosulfonat

0,91 g Estron, 1,73 g Bariumhydroxid, 0,089 g Cyclohexyltriethylammoniumbromid und 1,16 g N,N-Diethylamidosulfonylchlorid werden wie in Beispiel 1 angegeben in einem Gemisch von 5 ml tert.Amylalkohol und 2,5 ml Wasser zur Reaktion gebracht. Nach Aufarbeitung und Umkristallisation des Rohproduktes aus Methanol erhält man die Titelverbindung.
Fp.: 176-180 °C; [α]D: +109° (Chloroform, c=1)

### Beispiel 8 (= J 665): (Vergleichs beispiel) Herstellung von 17β-Hydroxy-19-nor-17α-pregna-1,3,5(10)-trien-20-in-3-yl N,N-dimethylamidosulfonat

1 g 17α-Ethinylestradiol, 2,4 g Natriumhydroxid, 0,24 g Triethylbenzylammoniumchlorid und 3,6 ml N,N-Dimethylamidosulfonylchlorid werden wie in Beispiel 1 beschrieben in einem Gemisch aus 30 ml Dichlormethan und 6,6 ml Wasser zur Reaktion gebracht. Nach Aufarbeitung, chromatographischer Reinigung und Umkristallisation des Reaktionsproduktes aus Aceton/n-Hexan erhält man die Titelverbindung. Fp.: 157-160 °C; [α]D: +4° (Chloroform, c=1)

### Beispiel 9 (= J 982):

### Herstellung von 17β-Hydroxy-14α,15α-methylen-estra-1,3,5(10)-trien-3-yl N,N-dimethylamidosulfonat

1 g 17β-Hydroxy-14α,15α-methylen-estra-1,3,5(10)-trien, 2,4 g Natriumhydroxid, 0,24 g Triethylbenzylammoniumchlorid und 3,6 ml N,N-Dimethylamidosulfonylchlorid werden wie in Beispiel 1 beschrieben in einem Gemisch aus 30 ml Dichlormethan und 6,6 ml Wasser zur Reaktion gebracht. Nach Aufarbeitung, chromatographischer Reinigung und Umkristallisation des Reaktionsproduktes aus Aceton erhält man die Titelverbindung.
Fp.: 193-196 °C; [α]D: +108° (Chloroform, c=1)

### Beispiel 10 (= J 984):

### Herstellung von 16α,17β-Dihydroxy-estra-1,3,5(10)-trien-3-yl N,N-diethylamidosulfonat

2 g Estriol, 5,2 g Natriumhydroxid, 1,72 g Triethylbenzylammoniumchlorid und 9,75 ml N,N-Diethylamidosulfonylchlorid werden wie in Beispiel 1 beschrieben in einem Gemisch aus 800 ml Toluol und 128 ml Wasser zur Reaktion gebracht. Nach Aufarbeitung, chromatographischer Reinigung und Umkristallisation aus Aceton erhält man die Titelverbindung.
Fp.: 121-124 °C; [α]D: +44° (Chloroform, c=1)

## Patentansprüche

1. Verwendung von Estra-1,3,5(10)-trien-Derivaten der allgemeinen Formel I 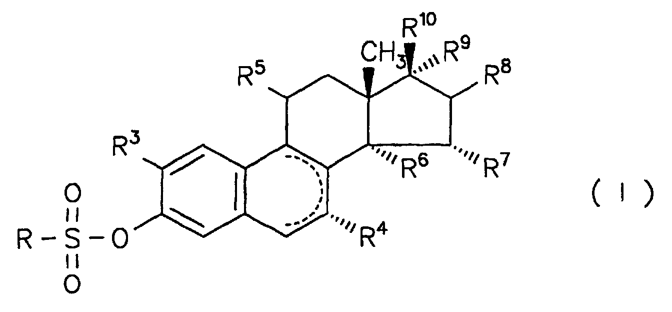 worin
R eine R¹R²N-Gruppe ist,
worin R¹ und R² unabhängig voneinander ein Wasserstoffatom, einen C₁-C₅-Alkylrest oder zusammen mit dem N-Atom einen Polymethyleniminorest mit 4 bis 6 C-Atomen oder einen Morpholinorest bedeuten,
R³ ein Wasserstoffatom, eine Hydroxygruppe oder eine Alkoxygruppe mit 1-5 C-Atomen bedeutet,
R⁴ ein Wasserstoffatom oder einen C₁-C₅-Alkylrest darstellt,
R⁵ ein Wasserstoffatom, eine Hydroxygruppe, eine veresterte Hydroxygruppe, eine Halogenalkyl- oder Alkoxy-gruppe mit 1-5 C-Atomen ist,
R⁶ und R⁷ Wasserstoffatome sind oder zusammen eine Methylengruppe darstellen,
R⁸, R⁹ und R¹⁰ unabhängig voneinander ein Wasserstoffatom oder eine Hydroxygruppe bedeuten
und der Ring B gegebenenfalls eine oder zwei Doppelbindungen enthält
oder
R⁹ und R¹⁰ zusammen ein Sauerstoffatom darstellen oder
R⁶ und R⁹ eine Vinylen- oder Ethylengruppe darstellen zur Herstellung von Arzneimitteln, die zur oralen Applikation geeignet sind, zur hormonalen Kontrazeption, zur Hormonsubstitutionstherapie und zur hormonalen Behandlung von Prostatakarzinomen.

2. Verwendung gemäß Anspruch 1, **dadurch gekennzeichnet, daß**
17β-Hydroxy-estra-1,3,5(10)-trien-3-yl-amidosulfonat,
17β-Hydroxy-estra-1,3,5(10)-trien-3-yl-N,N-diethylamidosulfonat,
17β-Hydroxy-estra-1,3,5(10)-trien-3-yl-N,N-dimethylamidosulfonat,
17β-Hydroxy-estra-1,3,5(10),7-tetraen-3-yl-N,N-diethylamidosulfonat,
17β-Hydroxy-estra-1,3,5(10),6,8-pentaen-3-yl-N,N-dimethylamidosulfonat,
17-Oxo-estra-1,3,5(10)-trien-3-yl-amidosulfonat,
17-Oxo-estra-1,3,5(10)-trien-3-yl-N,N-dimethylamidosulfonat,
16α,17β-Dihydroxy-estra-1,3,5(10)-trien-3-ylamidosulfonat,
16α,17β-Dihydroxy-estra-1,3,5(10)-trien-3-yl-N,N-dimethylamidosulfonat,
16α,17β-Dihydroxy-estra-1,3,5(10) -trien-3-yl-N,N-diethylamidosulfonat,
2,17β-Dihydroxy-estra-1,3,5(10)-trien-3-yl-N,N-dimethylamidosulfonat,
2-Methoxy-17β-hydroxy-estra-1,3,5(10)-trien-3-yl-N,N-diethylamidosulfonat,
17β-Hydroxy-14α,15α-methylen-estra-1,3,5(10)-trien-3-yl-N,N-dimethylamidosulfonat,
17β-Hydroxy-14α,15α-methylen-estra-1,3,5(10)-trien-3-yl-N,N-diethylamidosulfonat,
17β-Hydroxy-14α,15α-methylen-estra-1,3,5(10)-trien-3-yl-pyrrolidinosulfonat,
17β-Hydroxy-14α,15α-methylen-estra-1,3,5(10)-trien-3-yl-morpholinosulfonat,
17β-Hydroxy-14α,15α-methylen-estra-1,3,5(10)-trien-3-yl-N-methylamidosulfonat,
17β-Hydroxy-14α,15α-methylen-estra-1,3,5(10) -trien-3-yl-amidosulfonat,
17β-Hydroxy-14α,15α-methylen-estra-1,3,5(10),7-tetraen-3-yl-N,N-dimethylamidosulfonat,
17β-Hydroxy-14α,15α-methylen-estra-1,3,5(10) , 6,8-pentaen-3-yl-N,N-diethylamidosulfonat,
17β-Hydroxy-14α,15α-methylen-estra-1,3,5(10),8-tetraen-3-yl-N,N-dimethylamidosulfonat,
11β-Chlormethyl-17β-hydroxy-estra-1,3,5(10)-trien-3-yl-N,N-dimethylamidosulfonat,
17β-Hydroxy-14α,17α-vinylen-estra-1,3,5(10)-trien-3-yl-N,N-diethylamidosulfonat,
14α,17α-Ethylen-17β-hydroxy-estra-1,3,5(10)-trien-3-yl-pyrrolidinosulfonat,
16α,17β-Diydroxy-14α,17α-ethylen-estra-1,3,5(10)-trien-3-yl-N,N-diethylamidosulfonat und
17β-Hydroxy-7α-methyl-estra-1,3,5(10) -trien-3,11β-diyl-3-N,N-dimethylamidosulfonat-11-nitrat
verwendet werden.

3. Verwendung gemäß einem der Ansprüche 1 oder 2, **dadurch gekennzeichnet, daß** bei der oralen Applikation Tabletten, Tabletten mit kontrollierter Freisetzung, Dragees, Pillen, Kapseln, Filmtabletten und Filmtabletten mit kontrollierter Freisetzung verwendet werden.

## Claims

1. Use of estra-1,3,5(10)-triene derivatives of the general formula I 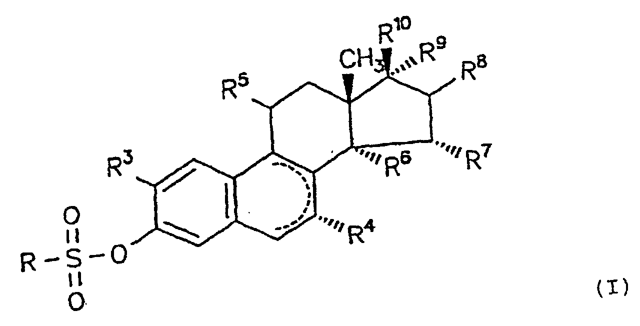 wherein
R is an R¹R²N group
wherein R¹ and R² are independent of each other and represent a hydrogen atom, a C₁ to C₅ alkyl radical, or together with the N atom
a polymethylene imino radical having 4 to 6 C atoms, or a morpholino radical,
R³ is a hydrogen atom, a hydroxy group, or an alkoxy group containing 1 to 5 C atoms,
R⁴ is a hydrogen atom or a C₁-C₅ alkyl radikal,
R⁵ is a hydrogen atom, a hydroxy group, an esterified hydroxy group, a haloalkyl or alkoxy group containing 1 to 5 C atoms,
R⁶ and R⁷ are hydrogen atoms, or together form a methylene group,
R⁸, R⁹ and R¹⁰ are independent of each other and represent a hydrogen atom or a hydroxy group,
and ring B optionally containing one or two double bondings or,
R⁹ and R¹⁰ together form an oxygen atom or
R⁶ and R⁹ represent a vinylene or ethylene group,
for the manufacture of drugs, which can be used for oral application for hormonal contraception, hormone replacement therapy and hormonal treatment of prostatic carcinomas.

2. Use according to claim 1, **characterized in that**
17β-hydroxy-estra-1,3,5(10)-trien-3-yl-amidosulfonate,
17β-hydroxy-estra-1,3,5(10)-trien-3-yl-N,N-diethylamidosulfonate,
17β-hydroxy-estra-1,3,5(10)-trien-3-yl-N,N-dimethylamidosulfonate,
17β-hydroxy-estra-1,3,5(10), 7-tetraene-3-yl-N,N-diethylamidosulfonate,
17β-hydroxy-estra-1,3,5(10), 6, 8-pentaene-3-yl-N,N-dimethylamidosulfonate,
17-oxo-estra-1,3,5(10)-trien-3-yl-amidosulfonate,
17-oxo-estra-1,3,5(10)-trien-3-yl-N,N-dimethylamidosulfonate,
16α, 17β-dihydroxy-estra-1,3,5(10)-trien-3-yl-amidosulfonate,
16α, 17β-dihydroxy-estra-1,3,5(10)-trien-3-yl-N,N-dimethylamidosulfonate,
16α, 17β-dihydroxy-estra-1,3,5(10)-trien-3-yl-N,N-diethylamidosulfonate,
2, 17β-dihydroxy-estra-1,3,5(10)-trien-3-yl-N,N-dimethylamidosulfonate,
2-methoxy-17β-hydroxy-estra-1,3,5(10)-trien-3-yl-N,N-diethylamidosulfonate,
17β-hydroxy-14α,15α-methylene-estra-1,3,5(10)-trien-3-yl-N,N-dimethylamidosulfonate,
17β-hydroxy-14α,15α-methylene-estra-1,3,5(10)-trien-3-yl-N,N-diethylamidosulfonate,
17β-hydroxy-14α,15α-methylene-estra-1,3,5(10)-trien-3-yl-pyrrolidinosulfonate,
17β-hydroxy-14α,15α-methylene-estra-1,3,5(10)-trien-3-yl-morpholinosulfonate,
17β-hydroxy-14α,15α-methylene-estra-1,3,5(10)-trien-3-yl-N-methylamidosulfonate,
17β-hydroxy-14α,15α-methylene-estra-1,3,5(10)-trien-3-yl-amidosulfonate,
17β-hydroxy-14α,15α-methylene-estra-1,3,5(10), 7-tetraene-3-yl-N,N-dimethylamidosulfonate,
17β-hydroxy-14α,15α-methylene-estra-1,3,5(10), 6,8-pentaene-3-yl-N,N-diethylamidosulfonate,
17β-hydroxy-14α,15α-methylene-estra-1,3,5(10), 8-tetraene-3-yl-N,N-dimethylamidosulfonate,
11β-chloromethyl-17β-hydroxy-estra-1,3,5(10)-trien-3-yl-N,N-dimethylamidosulfonate,
17β-hydroxy-14α,17α-vinylene-estra-1,3,5(10)-trien-3-yl-N,N-diethylamidosulfonate,
14α,17α-ethylene-17β-hydroxy-estra-1,3,5(10)-trien-3-yl-pyrrolidinosulfonate,
16α,17β-dihydroxy-14α,17α-ethylene-estra-1,3,5(10)-trien-3-yl-N,N-diethylamidosulfonate and
17β-hydroxy-7α-methyl-estra-1,3,5(10)-triene-3,11β-diyl-3-N,N-dimethylamidosulfonate-11-nitrate
are used.

3. Use according to claims 1 or 2, **characterized in that** for oral application tablets, tablets with controlled release, dragées, pills, capsules, film tablets, and film tablets with controlled release are used.

## Revendications

1. Utilisation de dérivés d'estra-1,3,5(10)-triène de formule générale I 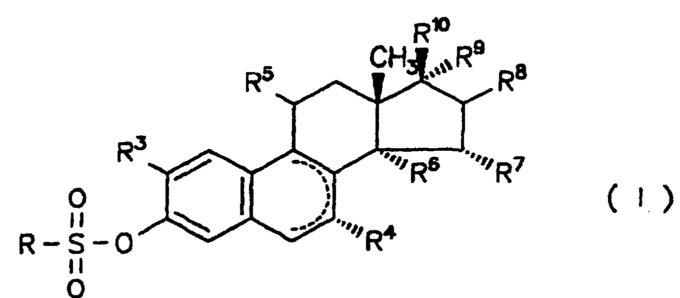 dans laquelle
R est un groupe R¹R²N, dans lequel
R¹ et R² signifient indépendamment l'un de l'autre un atome d'hydrogène, un radical alkyle en C₁-C₅, ou ensemble avec l'atome de N, signifient un radical polyméthylénimino comportant de 4 à 6 atomes de C ou un radical morpholino,
R³ représente un atome d'hydrogène, un groupe hydroxy ou un groupe alkoxy comportant de 1 à 5 atomes de C,
R⁴ représente un atome d'hydrogène ou un radical alkyle en C₁-C₅,
R⁵ représente un atome d'hydrogène, un groupe hydroxy, un groupe hydroxy estérifié, un groupe halogénoalkyle ou un groupe alkoxy comportant de 1 à 5 atomes de C,
R⁶ et R⁷ sont des atomes d'hydrogène ou représentent ensemble un groupe méthylène,
R⁸, R⁹ et R¹⁰ représentent indépendamment l'un de l'autre un atome d'hydrogène ou un groupe hydroxy
et le cycle B contient éventuellement une ou deux doubles liaisons
ou
R⁹ et R¹⁰ représentent ensemble un atome d'oxygène
ou
R⁶ et R⁹ représentent un groupe vinylène ou éthylène
pour la préparation de médicaments appropriés pour une application par voie orale destinés à la contraception hormonale, à la thérapie de substitution hormonale et au traitement hormonal des cancers de la prostate.

2. Utilisation selon la revendication 1, **caractérisée en ce qu'**on utilise les composés suivants :
17β-hydroxy-estra-1,3,5(10)-trién-3-yl-amidosulfonate,
17β-hydroxy-estra-1,3,5(10)-trién-3-yl-N,N-diéthylamidosulfonate,
17β-hydroxy-estra-1,3,5(10)-trién-3-yl-N,N-diméthylamidosulfonate,
17β-hydroxy-estra-1,3,5(10),7-tétraén-3-yl-N,N-diéthylamidosulfonate,
17β-hydroxy-estra-1,3,5(10),6,8-pentaén-3-yl-N,N-diméthylamidosulfonate,
17-oxo-estra-1,3,5(10)-tiién-3-yl-amidosulfonate,
17-oxo-estra-1,3,5(10)-trién-3-yl-N,N-diméthylamidosulfonate,
16α,17β-dihydroxy-estra-1,3,5(10)-trién-3-yl-amidosulfonate,
16α,17β-dihydroxy-estra-1,3,5(10)-trién-3-yl-N,N-diméthylamidosulfonate,
16α,17β-dihydroxy-estra-1,3,5(10)-trién-3-yl-N,N-diéthylamidosulfonate,
2,17β-dihydroxy-estra-1,3,5(10)-trién-3-yl-N,N-diméthylamidosulfonate,
2-méthoxy-17β-hydroxy-estra-1,3,5(10)-trién-3-yl-N,N-diéthylamidosulfonate,
17β-hydroxy-14α,15α-méthylén-estra-1,3,5(10)-trién-3-yl-N,N-diméthylamidosulfonate,
17β-hydroxy-14α,15α-méthylén-estra-1,3,5(10)-trién-3-yl-N,N-diéthylamidosulfonate,
17β-hydroxy-14α,15α-méthylén-estra-1,3,5(10)-trién-3-yl-pyrrolidlnosulfonate,
17β-hydroxy-14α,15α-méthylén-estra-1,3,5(10)-trién-3-yl-morpholinosulfonate,
17β-hydroxy-14α,15α-méthylén-estra-1,3,5(10)-trién-3-yl-N-méthylamidosulfonate,
17β-hydroxy-14α,15α-méthylén-estra-1,3,5(10)-trién-3-yl-amidosulfonate,
17β-hydroxy-14α,15α-méthylén-estra-1,3,5(10),7-tétraén-3-yl-N,N-diméthylamidosulfonate,
17β-hydroxy-14α,15α-méthylén-estra-1,3,5(10),6,8-pentaén-3-yl-N,N-diéthylamidosulfonate,
17β-hydroxy-14α,15α-méthylén-estra-1,3,5(10),8-tétraén-3-yl-N, N-diméthylamidosulfonate,
11β-chlornméthyl-17β-hydroxy-estra-1,3,5(10)-trién-3-yl-N,N-diméthylamidosulfonate,
17β-hydroxy-14α,17α-vinylén-estra-1,3,5(10)-trién-3-yl-N,N-diéthylamidosulfonate,
14α,17α-éthylén-17β-hydroxy-estra-1,3,5(10)-trién-3-yl-pyrrolidinosulfonate,
16α,17β-dihydroxy-14α,17α-éthylén-17β-hydroxy-estra-1,3,5( 10)-trién-3-yl-N,N-diéthylamidosulfonate et
17β-hydroxy-7α-méthyl-estra-1,3,5(10)-trién-3,11β-diyl-3-N,N-diméthylamidosulfonate-11-nitrate.

3. Utilisation selon une des revendications 1 ou 2, **caractérisée en ce qu'**en application par voie orale, on utilise des comprimés, des comprimés à libération contrôlée, des dragées, des pilules, des gélules, des comprimés enrobés d'un film et des comprimés enrobés d'un film à libération contrôlée.
